# EUROPEAN PATENT APPLICATION

(11) **EP 3 977 981 A1**
(43) Date of publication of application: **06.04.2022**
(21) Application number: 21188850.8
(22) Date of filing: 30.07.2021
(51) Int. Cl.: A61K 9/00, A61K 9/06, A61K 38/18, A61K 47/32, A61P 19/00

(54) **HYDROGEL FOR TOPICAL APPLICATION EFFECTIVE IN PREVENTING AND/OR ALLEVIATING CARTILAGE DEGENERATION**

(30) Priority: 05.08.2020 CH 9792020
(71) Applicant: Contrad Swiss SA, 6900 Lugano (CH)
(72) Inventor: SERRENTINO, Joanne, Sainte-Catherine-De-Hatley, JOB1WO (CA)
(74) Representative: Comoglio, Elena

(57) **Abstract**

The invention relates to a topical application hydrogel composition effective in relieving pain and preventing and/or attenuating cartilage degeneration, particularly in patients suffering from arthritis or osteoarthritis. The composition is characterized by a combination of biomimetic oligopeptides, as well as optionally by the presence of high molecular weight hyaluronic acid, panthenol, and one or more additional optional ingredients selected from the group consisting of thickening agents, wetting agents, solubilizing agents, chelating agents, pH adjusting agents, preservatives, and any combination thereof.

## Description

The present invention generally relates to the field of prevention and treatment of conditions characterized by reduced joint mobility. More precisely, the invention relates to a topical application hydrogel composition, which has a pain-relieving action and is effective in preventing and/or attenuating cartilage degeneration.

One class of active molecules still little used in this field are oligopeptides. In general terms, oligopeptides usually have a molecular weight of less than 5,000 Daltons and consist of amino acid chains that "mimic" the active parts of proteins normally found in the human body. Oligopeptides having these characteristics are called "biomimetic" peptides, since they can bind to specific cell receptors, and consequently to regulate a number of biological processes. One of the advantages of using oligopeptides in topical application compositions, in addition to their proven functionality, is their total safety of use, since they faithfully mimic parts of biomolecules normally found in the human body. The possibilities of developing biomimetic oligopeptides are countless, as well as the possibilities for the creation of a mix of oligopeptides with specific and, in any case, complementary actions.

The present invention provides a topical application hydrogel composition as defined in the appended claim 1, which has a combination of two particular biomimetic oligopeptides, said composition having proved highly functional for the well-being of cartilages.

The dependent claims define further features and advantages of the composition according to the invention.

The claims form an integral part of the specification.

As will be illustrated in detail in the following experimental section, thanks to its particular combination of active ingredients, as well as its pH and absorption characteristics, the composition of the invention was shown to be capable of temporarily restoring the viscoelasticity of the synovial fluid of the joints in the case of diseases characterized by cartilage damage and pain, such as arthritis and osteoarthritis, by protecting and slowing down the progression of joint damage and relieving the pain associated with these pathological conditions.

The composition of the invention comprises the combination of the following main components:
- a peptide component, consisting of the combination of a first oligopeptide comprising the amino acid sequence of SEQ ID NO:1 and a second oligopeptide comprising the amino acid sequence of SEQ ID NO:2;
- a gelling agent; and
- water (preferably demineralized water).

The first oligopeptide (designated as "SH-Polypeptide 85") is a single-chain recombinant human oligopeptide having a maximum length of 208 amino acids and comprising the amino acid sequence SEQ ID NO:1. It is manufactured through a recombinant technique known per se, including, for example, fermentation by a prokaryotic expression system, such as *E. coli,* by using a starting gene which is the synthetic copy of the human gene encoding for Fibroblast Growth Factor 9 (FGF9), used as such or adapted to the expression system used as the host.

The second oligopeptide (designated as "SH-Polypeptide 93") is a single-chain recombinant human oligopeptide having a maximum length of 349 amino acids and comprising the amino acid sequence SEQ ID NO:2. It is manufactured through a recombinant technique known per se, including, for example, fermentation by a prokaryotic expression system, such as *E. coli,* by using a starting gene which is the synthetic copy of the human gene encoding for the Cellular Communication Network factor 2 (CCN2), also designated as the connective tissue growth factor, used as such or adapted to the expression system used as the host.

The above oligopeptides may optionally comprise disulfide bridges and/or glycosylation.

SEQ ID NO:1: APLGEVGNYF GVQDAVPFGN VPVLPVDSPV LLSDHLGQSE

AGGLPRGPAV TDLDHLKGIL RRRQLYCRTG FHLEIFPNGT IQGTRKDHSR
FGILEFISIA VGLVSIRGVD SGLYLGMNEK GELYGSEKLT QECVFREQFE
ENWYNTYSSN LYKHVDTGRR YYVALNKDGT PREGTRTKRH QKFTHFLPRP
VDPDKVPELY KDILSQS

SEQ ID NO:2: MTAASMGPVR VAFVVLLALC SRPAVGQNCS GPCRCPDEPA
PRCPAGVSLV LDGCGCCRVC AKQLGELCTE RDPCDPHKGL FCDFGSPANR
KIGVCTAKDG APCIFGGTVY RSGESFQSSC KYQCTCLDGA VGCMPLCSMD
VRLPSPDCPF PRRVKLPGKC CEEWVCDEPK DQTVVGPALA AYRLEDTFGP
DPTMIRANCL VQTTEWSACS KTCGMGISTR VTNDNASCRL EKQSRLCMVR
PCEADLEENI KKGKKCIRTP KISKPIKFEL SGCTSMKTYR AKFCGVCTDG
RCCTPHRTTT LPVEFKCPDG EVMKKNMMFI KTCACHYNCP GDNDIFESLY
YRKMYGDMA

According to a preferred embodiment, SH-Polypeptide 85 and SH-Polypeptide 93 consist of the amino acid sequences SEQ ID Nos.: 1 and 2, respectively, shown above.

The gelling agent used in the composition of the present invention is a rheology modifier capable of changing a mixture from the liquid to the gel phase. Gelling agents particularly suitable for use in the composition of the invention, which is water-based, are acrylic polymers and derivatives thereof, such as for example "Carbopols", i.e., a term which identifies a family of high-molecular-weight cross-linked polyacrylic acid polymers. A particularly preferred gelling agent within the scope of the present invention is Carbopol^{®} Ultrez 30.

In a preferred embodiment, the composition of the invention also contains high molecular weight (1000 - 3000 kDaltons) hyaluronic acid. This substance has remarkable hygroscopic characteristics and assists in improving the skin's barrier function and consequently skin moisturization, i.e., a useful feature in topical application compositions.

In another preferred embodiment, the composition of the invention also contains panthenol (provitamin B5), i.e., a vitamin B5 precursor which contributes to a soothing and calming action on the skin; it can therefore be considered a valid support to the regenerating activity performed by the combination of oligopeptides defined above.

According to further preferred embodiments, the composition of the present invention also contains one or more of the following additional components: thickening agents, wetting agents, solubilizing agents, chelating agents, pH adjusting agents, and preserving agents.

An exemplary thickening agent suitable for use in the invention is xanthan gum (for example marketed as Rheocare^{®} XGN), which is a high molecular weight polysaccharide with a thickening and stabilizing action on the gel form.

Exemplary wetting agents suitable for use in the invention are glycerin and propylene glycol; the latter is a so-called "skin enhancer" or "skin carrier", as it enhances the absorption of the product, and therefore also of its main functional ingredients into the skin.

An exemplary solubilizing agent suitable for use in the invention is PEG-40 hydrogenated castor oil. This ingredient is useful for optimally solubilizing the ingredients of the composition, thus avoiding the risk of separation and chemical-physical instability.

Exemplary preservatives suitable for use in the invention are dehydroacetic acid, benzoic acid, ethylhexylglycerin and phenoxyethanol; particularly if used in a mixture (for example marketed as Euxyl^{®} K701), they can prevent any type of bacterial and/or fungal contamination of the composition.

An exemplary chelating agent suitable for use in the invention is ethylenediaminetetraacetic acid (EDTA) disodium salt, which is suitable to assist the preservative system by acting as a heavy metal chelator.

An exemplary pH adjusting agent suitable for use in the composition of the invention is sodium hydroxide, which is introduced in quantities such as to adjust the pH of the composition within the identified preferred range (pH between 5.00 and 5.70).

According to a preferred embodiment, the composition of the invention does not contain perfuming substances, therefore it does not contain any perfume allergens.

The preferred embodiments described above can be combined with each other as required, and the implementation of such combinations falls within the skills of those of ordinary skill in the art.

Table 1 below illustrates, by way of non-limiting example, suitable percentages (% w/w) of the ingredients of a composition falling within the scope of the present invention:

**Table 1**

| **Ingredient** | **% w/w** |
|---|---|
| Demineralized water | >75 |
| Glycerin^{®} 99.8% pharmaceutical grade | 1-5 |
| HMW Hyaluronic acid | 0.1-1 |
| Rheocare XGN | 0.1-1 |
| Disodium EDTA | 0.1-1 |
| Carbopol Ultrez 30 | 1-5 |
| 30% Sodium hydroxide solution | <0.1 |
| PEG 40 hydrogenated castor oil | 0.1-1 |
| Euxyl K701 | 0.1-1 |
| Monopropylene glycol | 0.1-1 |
| Panthenol D USP | 0.1-1 |
| SH-Polypeptide 85 | 0.0000001-0.1 |
| SH-Polypeptide 93 | 0.0000001-0.1 |

The composition of Table 1 was packaged as single doses.

The composition of the present invention is a hydrogel based on oligopeptides and hyaluronic acid with a soothing action, useful to prevent and attenuate the physiological degeneration of cartilage, by protecting and slowing down the progression of joint damage. It allows the viscoelasticity of the synovial fluid of the joints to be temporarily restored in case of problems due to diseases such as arthritis and osteoarthritis, through the action of hyaluronic acid, fibroblast growth factor 9 (FGF-9) and cellular communication network factor 2 (CCN2).

The cellular and biochemical role of hyaluronic acid (HA) depends on its molecular weight; the high molecular weight (HMW) HA fraction has been shown to possess antiinflammatory properties [1] through the regulation of pro-inflammatory genes [2] and the action of inflammatory cells [3]. Resolving inflammation can help promote healing and new tissue formation and can help reduce fibrosis and scarring [4]. In osteoarthritis (OA), inflammation is associated with disease progression and worse symptoms [5].

The viscoelasticity of the synovial fluid is mainly due to the presence of hyaluronic acid (HA) [6]. Genetic ablation of CCN2 has been shown to reduce the production of HA and other extracellular matrix proteins, as well as to reduce circulating levels of HA, a key modulator of viscoelasticity [7], [8].

The role of FGF9 in joint development and homeostasis is well established, particularly its role in controlling chondrocyte development and regulating bone growth [9]-[11], which are factors that contribute to smooth, and therefore painless joint movement.

### Experimental Section

A composition prepared with the ingredients described in Table 1 and in amounts falling within the quantitative ranges described therein was subjected to a case study aimed at assessing the effectiveness of the product in helping to prevent and attenuate cartilage physiological degeneration in osteoarthritis, thus protecting and slowing down the progression of joint damage in a 51-year-old woman with an osteoarthritic knee joint with collateral ligament sprain. Infrared (IR) images were taken which revealed the presence of synovial inflammation. The images showed no inflammation in the contralateral limb.

Several measurements of cartilage, pain, and mobility were made before and after treatment with the composition in order to assess the effectiveness of the composition of the invention.

### 1. KOOS (knee injury and osteoarthritis outcome score)

The KOOS questionnaire was developed to assess short- and long-term symptoms and function in subjects with knee injury and osteoarthritis. KOOS assesses both short-term and long-term consequences of knee injury. It contains 42 items in 5 separately scored subscales: pain, other symptoms, activities in daily living (ADL), function in sports and recreation (sports, etc.), and knee-related quality of life (QOL) [12]. Scores are transformed into a 0-100 scale, where zero represents "extreme problems" of the knee and 100 represents "no problems" of the knee. A change of 8-10 points on the KOOS scale corresponds to a clinically significant improvement [12].

The KOOS questionnaire was completed within 24 hours of the injury and prior to treatment of the affected joint with the composition of the invention, after two weeks and four applications of the single-dose composition, and again after four weeks and a total of nine applications of the single-dose composition.

### 2. LYSHOLM

The Lysholm questionnaire is a system for examining specific symptoms in a patient's knee, including lameness, use of supports, joint locking, feeling unstable, pain, swelling, ability to climb stairs and difficulty in squatting. It has been validated as a patient-administered tool for measuring symptoms and function in patients with a variety of knee injuries [13]. Eight factors are rated to produce an overall score on a scale of 0 to 100. A lower score represents a higher degree of pain and disability. In particular, a score from 95 to 100 is regarded as "excellent"; a score from 84 to 94 is regarded as "good"; a score from 65 to 83 is regarded as "fair", and a score of less than 65 is regarded as "poor". Factors such as lameness, use of supports, and joint locking are worth a potential of 23 points; pain and instability, 25 points each; swelling and ability to climb stairs, 10 points each; and ability to remain squatted, 5 points [14].

The Lysholm questionnaire was completed within 24 hours of the injury and prior to treatment of the affected joint with the composition of the invention, after two weeks and four applications of the single-dose composition, and again after four weeks and a total of nine applications of the single-dose composition.

### 3. Infrared imaging (IR)

Digital infrared imaging was used to assess changes in connective tissue matrix density. Mid-infrared (MIR) spectroscopic assessment of cartilage is well established, with many studies validating the assignment of specific bands present in MIR spectra to specific molecular vibrations [15]. In this way, MIR can be used to identify the collagen and proteoglycan content of the analysed tissue [16]. Measurements were obtained by using an infrared camera (ICI DIGITAL THERMAL version TEi P V6. 8), with a spectral sensitivity of 8-12 µm at a geometric resolution of 1.5 mrad and a field of view of 30-20°. The focusing distance was <20 cm, the thermal resolution was 0.03 K, and the emissivity value was 0.98. The frame rate was set at 60 Hz. "ICI IR flash pro reporter" software, 75% and 50% grey option, was used.

The IR images were taken within 24 hours of the injury, prior to treatment of the affected joint with the composition of the invention. Further images were taken after two weeks, during which four single doses of the composition of the invention were applied to the patient, and again after four weeks and a total of nine single-dose applications of the composition of the invention.

The results obtained suggest that the composition of the invention resulted in an increase in the knee's soft tissue, with a decrease in pain and disability in the affected area after four weeks during which nine single doses of the product were applied.

Prior to treatment, the patient had a significant level of disability, as shown by the KOOS (39/100) and Lysholm (45/100) scores.

After two weeks, during which the composition was applied four times, both the KOOS score and the Lysholm score increased on average by 26 points, indicating a clinically significant reduction in the level of disability and an increase in the patient's range of motion. The IR images acquired after two weeks revealed the presence of degraded cartilage areas, characterized by non-crosslinked extracellular matrix and/or collagen fibres, which can be identified as dark blue vertical bars in the IR images.

After four weeks of treatment with the composition of the invention, during which a total of nine single doses were applied to the patient, a further marked improvement in KOOS and Lysholm questionnaires was found, with further reductions in disability and increase in the range of motion. Evidence of collagen fibres and/or degraded/non-crosslinked extracellular matrix was reduced in the IR images. This is indicative of the deposition of new tissue from the extracellular matrix, i.e., collagen fibres and cartilage. An increase in tissue density caused by growth and formation of new cartilage and collagen was also found after four weeks of treatment. These thermographic results are related to the improvements found with both questionnaires.

In particular, the following was observed:
- A reduction in the disability status, highlighted by the increase in the KOOS score from a value of 39 before applying the product to a value of 70 after two weeks. A further increase in the KOOS score up to 91 was detected after four weeks (Table 2).
- A reduction in the symptoms, highlighted by the increase in the Lysholm score from a value of 45 before applying the product to a value of 66 after two weeks. A further increase in the KOOS score up to 80 was detected after four weeks (Table 2).
- A sharp increase in the thickness of the trochlear groove after applying the product, with an increase in tissue density determined by cartilage formation and cross-linked collagen fibres.

**Table 2. Results from the KOOS and Lysholm questionnaires to quantify the patient's disability status. In both cases, a low score indicates high disability.**

| | KOOS (0-100) | LYSHOLM (0-100) |
|---|---|---|
| Prior to application | 39 | 45 |
| After two weeks | 70 | 66 |
| After four weeks | 91 | 80 |

### References

[1] G. D. Albano et al., 'Effect of High, Medium, and Low Molecular Weight Hyaluronan on Inflammation and Oxidative Stress in an In Vitro Model of Human Nasal Epithelial Cells', Mediators of Inflammation, 2016. https://www.hindawi.com/journals/mi/2016/8727289/ (accessed Nov. 18, 2019).
[2] D. Jiang et al., 'Regulation of lung injury and repair by Toll-like receptors and hyaluronan', Nat. Med., vol. 11, no. 11, pp. 1173-1179, Nov. 2005, doi: 10.1038/nm1315.
[3] P. L. Bollyky et al., 'ECM components guide IL-10 producing regulatory T-cell (TR1) induction from effector memory T-cell precursors', Proc. Natl. Acad. Sci. U.S.A., vol. 108, no. 19, pp. 7938-7943, May 2011, doi: 10.1073/pnas.1017360108.
[4] J. Larouche, S. Sheoran, K. Maruyama, and M. M. Martino, 'Immune Regulation of Skin Wound Healing: Mechanisms and Novel Therapeutic Targets', Adv Wound Care (New Rochelle), vol. 7, no. 7, pp. 209-231, Jul. 2018, doi: 10.1089/wound.2017.0761.
[5] J. Lieberthal, N. Sambamurthy, and C. R. Scanzello, 'Inflammation in Joint Injury and Post-Traumatic Osteoarthritis', Osteoarthritis Cartilage, vol. 23, no. 11, pp. 1825-1834, Nov. 2015, doi: 10.1016/j.joca.2015.08.015.
[6] M. Kawata, A. Okamoto, T. Endo, and Y. Tsukamoto, '- Viscoelasticity of synovial fluids and additive effect of hyaluronate', in Hydrocolloids, K. Nishinari, Ed. Amsterdam: Elsevier Science, 2000, pp. 343-348.
[7] Z. Yuhua, R. Wanhua, S. Chenggang, S. Jun, W. Yanjun, and Z. Chunqing, 'Disruption of connective tissue growth factor by short hairpin RNA inhibits collagen synthesis and extracellular matrix secretion in hepatic stellate cells', Liver Int., vol. 28, no. 5, pp. 632-639, May 2008, doi: 10.1111/j.1478-3231.2008.01730.x.
[8] Z. Tong, R. Chen, D. S. Alt, S. Kemper, B. Perbal, and D. R. Brigstock, 'Susceptibility to liver fibrosis in mice expressing a connective tissue growth factor transgene in hepatocytes', Hepatology, vol. 50, no. 3, pp. 939-947, 2009, doi: 10.1002/hep.23102.
[9] S. Zhou et al., 'Exogenous fibroblast growth factor 9 attenuates cartilage degradation and aggravates osteophyte formation in post-traumatic osteoarthritis', Osteoarthritis and Cartilage, vol. 24, no. 12, pp. 2181-2192, Dec. 2016, doi: 10.1016/j.joca.2016.07.005.
[10] X. Wu et al., 'Multiple Synostoses Syndrome Is Due to a Missense Mutation in Exon 2 of FGF9 Gene', The American Journal of Human Genetics, vol. 85, no. 1, pp. 53-63, Jul. 2009, doi: 10.1016/j.ajhg.2009.06.007.
[11] D. M. Ornitz and N. Itoh, 'The Fibroblast Growth Factor signaling pathway', WIREs Developmental Biology, vol. 4, no. 3, pp. 215-266, 2015, doi: 10.1002/wdev.176.
[12] E. M. Roos and L. S. Lohmander, 'The Knee injury and Osteoarthritis Outcome Score (KOOS): from joint injury to osteoarthritis', Health Qual Life Outcomes, vol. 1, p. 64, Nov. 2003, doi: 10.1186/1477-7525-1-64.
[13] M. S. Kocher, J. R. Steadman, K. K. Briggs, W. I. Sterett, and R. J. Hawkins, 'Reliability, validity, and responsiveness of the Lysholm knee scale for various chondral disorders of the knee', J Bone Joint Surg Am, vol. 86, no. 6, pp. 1139-1145, Jun. 2004, doi: 10.2106/00004623-200406000-00004.
[14] S. D. Barber-Westin and F. R. Noyes, '43 - Rating of Athletic and Daily Functional Activities: Knee-Specific Scales and Global Outcome Instruments', in Noyes' Knee Disorders: Surgery, Rehabilitation, Clinical Outcomes (Second Edition), F. R. Noyes and S. D. Barber-Westin, Eds. Elsevier, 2017, pp. 1211-1221.
[15] U. P. Palukuru, A. Hanifi, C. M. McGoverin, S. Devlin, P. I. Lelkes, and N. Pleshko, 'Near infrared spectroscopic imaging assessment of cartilage composition: Validation with mid infrared imaging spectroscopy', Anal Chim Acta, vol. 926, pp. 79-87, Jul. 2016, doi: 10.1016/j.aca.2016.04.031.
[16] K. W. Sanden et al., 'The use of Fourier-transform infrared spectroscopy to characterize connective tissue components in skeletal muscle of Atlantic cod (Gadus morhua L.)', Journal of Biophotonics, vol. 12, no. 9, p. e201800436, 2019, doi: 10.1002/jbio.201800436.

## Claims

1. A topical application hydrogel composition comprising:
- a peptide component, consisting of the combination of a first oligopeptide comprising the amino acid sequence of SEQ ID NO:1 and a second oligopeptide comprising the amino acid sequence of SEQ ID NO:2;
- a gelling agent; and
- water.

2. The topical application hydrogel composition according to claim 1, wherein the gelling agent is a high molecular weight cross-linked polyacrylic acid polymer.

3. The topical application hydrogel composition according to claim 1 or 2, further comprising high molecular weight hyaluronic acid.

4. The topical application hydrogel composition according to any one of claims 1 to 3, further comprising panthenol.

5. The topical application hydrogel composition according to any one of claims 1 to 4, also comprising further ingredients selected from the group consisting of thickening agents, wetting agents, solubilizing agents, chelating agents, pH adjusting agents, preserving agents, and any combination thereof.

6. The topical application hydrogel composition according to claim 5, comprising xanthan gum as the thickening agent.

7. The topical application hydrogel composition according to claim 5 or 6, comprising glycerin and propylene glycol as the wetting agent.

8. The topical application hydrogel composition according to any one of claims 5 to 7, comprising PEG-40 hydrogenated castor oil as the solubilizing agent.

9. The topical application hydrogel composition according to any one of claims 5 to 8, comprising dehydroacetic acid, benzoic acid, ethylhexylglycerin and phenoxyethanol as the preservative agents.

10. The topical application hydrogel composition according to any one of claims 5 to 9, comprising EDTA disodium salt as the chelating agent.

11. The topical application hydrogel composition according to any one of claims 5 to 10, comprising sodium hydroxide as the pH adjusting agent.

12. The topical application hydrogel composition according to any one of claims 1 to 11, having a pH between 5.00 and 5.70.

13. The topical application hydrogel composition as defined in any one of claims 1 to 12, packaged in the form of one or more single doses.

14. The topical application hydrogel composition as defined in any one of claims 1 to 13, for use in relieving pain and preventing and/or attenuating cartilage degeneration.

15. The topical application hydrogel composition for use in relieving pain and preventing and/or attenuating cartilage degeneration in a patient suffering from arthritis or osteoarthritis.


SEQUENCE LISTING

<110> Contrad Swiss SA

<120> Idrogel ad applicazione topica efficace nel prevenire e/o attenuare la
degenerazione della cartilagine

<130> E0135610-E6675-EC

<160> 2

<170> BiSSAP 1.3.6

<210> 1
<211> 207
<212> PRT
<213> Homo sapiens

<400> 1
Ala Pro Leu Gly Glu Val Gly Asn Tyr Phe Gly Val Gln Asp Ala Val
1               5                   10                  15
Pro Phe Gly Asn Val Pro Val Leu Pro Val Asp Ser Pro Val Leu Leu
            20                  25                  30
Ser Asp His Leu Gly Gln Ser Glu Ala Gly Gly Leu Pro Arg Gly Pro
        35                  40                  45
Ala Val Thr Asp Leu Asp His Leu Lys Gly Ile Leu Arg Arg Arg Gln
    50                  55                  60
Leu Tyr Cys Arg Thr Gly Phe His Leu Glu Ile Phe Pro Asn Gly Thr
65                  70                  75                  80
Ile Gln Gly Thr Arg Lys Asp His Ser Arg Phe Gly Ile Leu Glu Phe
                85                  90                  95
Ile Ser Ile Ala Val Gly Leu Val Ser Ile Arg Gly Val Asp Ser Gly
            100                 105                 110
Leu Tyr Leu Gly Met Asn Glu Lys Gly Glu Leu Tyr Gly Ser Glu Lys
        115                 120                 125
Leu Thr Gln Glu Cys Val Phe Arg Glu Gln Phe Glu Glu Asn Trp Tyr
    130                 135                 140
Asn Thr Tyr Ser Ser Asn Leu Tyr Lys His Val Asp Thr Gly Arg Arg
145                 150                 155                 160
Tyr Tyr Val Ala Leu Asn Lys Asp Gly Thr Pro Arg Glu Gly Thr Arg
                165                 170                 175
Thr Lys Arg His Gln Lys Phe Thr His Phe Leu Pro Arg Pro Val Asp
            180                 185                 190
Pro Asp Lys Val Pro Glu Leu Tyr Lys Asp Ile Leu Ser Gln Ser
        195                 200                 205

<210> 2
<211> 349
<212> PRT
<213> Homo sapiens

<400> 2
Met Thr Ala Ala Ser Met Gly Pro Val Arg Val Ala Phe Val Val Leu
1               5                   10                  15
Leu Ala Leu Cys Ser Arg Pro Ala Val Gly Gln Asn Cys Ser Gly Pro
            20                  25                  30
Cys Arg Cys Pro Asp Glu Pro Ala Pro Arg Cys Pro Ala Gly Val Ser
        35                  40                  45
Leu Val Leu Asp Gly Cys Gly Cys Cys Arg Val Cys Ala Lys Gln Leu
    50                  55                  60
Gly Glu Leu Cys Thr Glu Arg Asp Pro Cys Asp Pro His Lys Gly Leu

65                  70                  75                  80
Phe Cys Asp Phe Gly Ser Pro Ala Asn Arg Lys Ile Gly Val Cys Thr
                85                  90                  95
Ala Lys Asp Gly Ala Pro Cys Ile Phe Gly Gly Thr Val Tyr Arg Ser
            100                 105                 110
Gly Glu Ser Phe Gln Ser Ser Cys Lys Tyr Gln Cys Thr Cys Leu Asp
        115                 120                 125
Gly Ala Val Gly Cys Met Pro Leu Cys Ser Met Asp Val Arg Leu Pro
    130                 135                 140
Ser Pro Asp Cys Pro Phe Pro Arg Arg Val Lys Leu Pro Gly Lys Cys
145                 150                 155                 160
Cys Glu Glu Trp Val Cys Asp Glu Pro Lys Asp Gln Thr Val Val Gly
                165                 170                 175
Pro Ala Leu Ala Ala Tyr Arg Leu Glu Asp Thr Phe Gly Pro Asp Pro
            180                 185                 190
Thr Met Ile Arg Ala Asn Cys Leu Val Gln Thr Thr Glu Trp Ser Ala
        195                 200                 205
Cys Ser Lys Thr Cys Gly Met Gly Ile Ser Thr Arg Val Thr Asn Asp
    210                 215                 220
Asn Ala Ser Cys Arg Leu Glu Lys Gln Ser Arg Leu Cys Met Val Arg
225                 230                 235                 240
Pro Cys Glu Ala Asp Leu Glu Glu Asn Ile Lys Lys Gly Lys Lys Cys
                245                 250                 255
Ile Arg Thr Pro Lys Ile Ser Lys Pro Ile Lys Phe Glu Leu Ser Gly
            260                 265                 270
Cys Thr Ser Met Lys Thr Tyr Arg Ala Lys Phe Cys Gly Val Cys Thr
        275                 280                 285
Asp Gly Arg Cys Cys Thr Pro His Arg Thr Thr Thr Leu Pro Val Glu
    290                 295                 300
Phe Lys Cys Pro Asp Gly Glu Val Met Lys Lys Asn Met Met Phe Ile
305                 310                 315                 320
Lys Thr Cys Ala Cys His Tyr Asn Cys Pro Gly Asp Asn Asp Ile Phe
                325                 330                 335
Glu Ser Leu Tyr Tyr Arg Lys Met Tyr Gly Asp Met Ala
            340                 345